Europäisches Patentamt

European Patent Office

Office européen des brevets

(19)

(11) **EP 1 129 992 A1**

(12) **EUROPÄISCHE PATENTANMELDUNG**

(43) Veröffentlichungstag:
**05.09.2001 Patentblatt 2001/36**

(51) Int Cl.[7]: **C01B 37/00**, B01J 29/04,
C07D 301/12

(21) Anmeldenummer: **00104325.6**

(22) Anmeldetag: **02.03.2000**

(84) Benannte Vertragsstaaten:
**AT BE CH CY DE DK ES FI FR GB GR IE IT LI LU MC NL PT SE**
Benannte Erstreckungsstaaten:
**AL LT LV MK RO SI**

(71) Anmelder: **Degussa AG**
**40474 Düsseldorf (DE)**

(72) Erfinder: **Hasenzahl, Steffen**
**63477 Maintal (DE)**

(74) Vertreter:
**Sternagel, Fleischer, Godemeyer & Partner Patentanwälte**
**Braunsberger Feld 29**
**51429 Bergisch Gladbach (DE)**

(54) **Verfahren zur Herstellung eines titanhaltigen Zeolithen**

(57) Die vorliegende Erfindung betrifft ein Verfahren zur Herstellung eines titanhaltigen Zeolithen durch

a) Zusammengeben einer hydrolisierbaren Siliciumverbindung und einer hydrolisierbaren Titanverbindung,

b) Zugeben einer basischen quartären Ammoniumverbindung in wäßrigem Medium zu der Mischung aus a) und Hydrolisieren der Reaktionsmischung bei einer Temperatur im Bereich von 0°C - 100° zur Ausbildung eines Synthesesols, anschließend

c) Erwärmen der Synthesesols auf eine Temperatur im Bereich von 150°C bis 190°C und

d) Kristallisieren des Synthesesols bei dieser Temperatur,

dadurch gekennzeichnet, daß
die Aufheizzeit in Schritt c) weniger als 240 Minuten beträgt.

EP 1 129 992 A1

**Beschreibung**

Die vorliegende Erfindung betrifft ein Verfahren zur Herstellung eines titanhaltigen Zeolithen sowie die Verwendung eines so erhältlichen Zeolithen als Katalysator für die Epoxidierung von Olefinen mit Wasserstoffperoxid und ein Verfahren zur Epoxidierung von Olefinen in Gegenwart eines gemäß des erfindungsgemäßen Verfahrens hergestellten titanhaltigen Zeolithen.

Aus US-A 4,410,501 ist ein Verfahren zur Herstellung von Titansilicalit wie auch die Verwendung des so hergestellten Titansilicalits als Katalysator in einer Reihe von Reaktionen, u. a. auch Oxidationsreaktionen, bekannt. Hierin sind zwei verschiedene Verfahrensweisen beschrieben, u. a. die Ausbildung eines Synthesegels, ausgehend von einer hydrolysierbaren Siliciumverbindung wie z. B. Tetraethylorthosilicat und einer hydrolysierbaren Titanverbindung durch Zugabe von Tetra-n-propylammoniumhydroxid (TPAOH) und Hydrolysieren dieser Reaktionsmischung.

In den folgenden Jahren haben viele Forschergruppen in Industrie und Universitäten versucht, ausgehend von der Lehre der US-A 4,410,501 die Synthese von Titansilicalit sowohl bezüglich der Aktivität des resultierenden Katalysators als auch bezüglich der Verfahrenseffizienz zu optimieren.

So wurde in verschiedenen wissenschaftlichen Veröffentlichungen (A. J. H. van der Pol und J. H. C. van Hooff, Applied Catalysis A: General, 92 (1992) 93-100; van der Pol, Verduyn und van Hooff, Applied Catalysis A: General, 92 (1992) 113-130; J. A. Martens et al., Applied Catalysis A: General, 93 (1993) 71-84) der Einfluß einer Vielzahl von Prozeßparametern wie z. B. Art der $SiO_2$-Quelle, Kristallisationsdauer, Kristallisationsbedingungen, Verhältnis von Templatverbindung/Silicium und Silicium/Titan in den Ausgangsstoffen auf die Aktivität des resultierenden Katalysators untersucht. Trotz einer sehr weitgehenden und tiefgehenden Auseinandersetzung mit den Parametern, die die Synthese und Aktivität von Titansilicalitkatalysatoren beeinflussen, besteht dennoch nach wie vor ein Bedürfnis in der Industrie, das Herstellungverfahren bezüglich der Aktivität des resultierenden Katalysators weiter zu verbessern.

Die Aufgabe, die der vorliegenden Erfindung somit zugrunde liegt besteht darin, ausgehend von dem oben beschriebenen Stand der Technik die Synthese von titanhaltigen Zeolithen weiter zu verbessern, um die Aktivität des resultierenden Katalysators, insbesondere für die Epoxidierung von Olefinen zu steigern.

Diese Aufgabe wird durch ein Verfahren zur Herstellung eines titanhaltigen Zeolithen durch

> a) Zusammengeben einer hydrolysierbaren Siliciumverbindung und einer hydrolysierbaren Titanverbindung,
> b) Zugeben einer basischen quartären Ammoniumverbindung in wäßrigem Medium zu der Mischung aus a) und Hydrolysieren der Reaktionsmischung bei einer Temperatur im Bereich von 0°C - 100°C zur Ausbildung eines Synthesesols, anschließend
> c) Erwärmen des Synthesesols auf eine Temperatur im Bereich von 150°C - 190°C,
> d) Kristallisieren des Synthesesols bei dieser Temperatur, dadurch gekennzeichnet, daß die Aufheizzeit in Schritt c) weniger als 240 min beträgt,

gelöst.

Entsprechend einer bevorzugten Ausführungsform liegt die Aufheizzeit im Schritt c) zwischen 60 und 180 min, vorzugsweise zwischen 80 und 150 min, wobei eine Aufheizzeit von 90 bis 120 min am meisten bevorzugt ist.

Entsprechend der vorliegenden Erfindung werden zunächst eine hydrolysierbare Siliciumverbindung und eine hydrolysierbare Titanverbindung mit einer basischen quartären Ammoniumverbindung in Gegenwart von Wasser hydrolysiert. Als hydrolysierbare Silicium, bzw. Titanverbindungen eignen sich insbesondere für das erfindungsgemäße Verfahren die Tetraalkylorthosilicate bzw. Tetraalkylorthotitanate wobei Alkyl vorzugsweise ausgewählt ist aus der Gruppe bestehend aus Methyl, Ethyl, Propyl oder Butyl. Die am meisten bevorzugten Ausgangsverbindungen sind Tetraethylorthosilicat und Tetraethylorthotitanat, wobei Alkyl vorzugsweise ausgewählt ist aus der Gruppe bestehend aus Methyl, Ethyl, Propyl oder Butyl. Die am meisten bevorzugten Ausgangsverbindungen sind Tetraethylorthosilicat und Tetraethylorthotitanat.

Bei der quartären Ammoniumverbindung handelt es sich um eine Templatverbindung, die durch Aufnahme in das Kristallgitter des Produkts während der Kristallisation die Kristallstruktur bestimmt. Bevorzugt werden Tetraalkylammoniumverbindungen wie Tetraalkylammoniumhydroxid, insbesondere Tetra-n-propylammoniumhydroxid zur Herstellung von Titansilicalit-1 (MFI-Struktur), Tetra-n-butyl-ammoniumhydroxid zur Herstellung von Titansilicalit-2 (MEL-Struktur) und Tetraethylammoniumhydroxid zur Herstellung von Titan-β-Zeolith (BEA-Kristallstruktur) eingesetzt. Die quartäre Ammoniumverbindung wird vorzugsweise als wäßrige Lösung eingesetzt.

Der für die Synthese erforderliche pH-Wert des Synthesesols von > 9, bevorzugt > 11, stellt sich durch die basisch reagierende quartäre Ammoniumverbindung ein.

Die Temperatur, bei der das Synthesesol hergestellt wird, kann in weiten Grenzen gewählt werden, bevorzugt ist allerdings das Gemisch aus hydrolysierbarer Siliciumverbindung und hydrolysierbarer Titanverbindung auf eine Temperatur im Bereich von 0°C bis 10°C, vorzugsweise 0°C bis 5°C, besonders bevorzugt 1°C abzukühlen und dann die basische quartäre Ammoniumverbindung in wäßriger Lösung, die auf die gleiche Temperatur abgekühlt ist, zuzugeben.

In einer weiteren Ausführungsform der vorliegenden Erfindung wird bei der Verwendung von Tetraalkylorthosilicaten und Tetraalkylorthotitanaten als Silicium- bzw. Titanquelle nach Schritt b) und vor Schritt c) des erfindungsgemäßen Verfahrens das Synthesesol auf eine Temperatur von 75°C bis 95°C für eine Zeitdauer von 120 bis 200 min erwärmt und der entstehende Alkohol als Wasser-Azeotrop abdestilliert, um die Hydrolysierung der hydrolysierbaren Titan- und Siliciumverbindung zu unterstützen. Üblicherweise wird das durch Abdestillieren entnommene Volumen an Alkohol/Wasser-Azeotrop zumindest teilweise in der Reaktionsmischung durch Wasser ersetzt, um die Ausbildung eines festen Gels bzw. Wandablagerungen während der Kristallisation zu vermeiden.

Das nach Beendigung der Hydrolysierung entstandene Synthesesol wird dann erfindungsgemäß innerhalb eines weiter oben definierten Zeitraums auf die Kristallisationstemperatur von 150°C bis 190°C erwärmt. Überraschenderweise wurde gefunden, daß die Aktivität des resultierenden Katalysators von der Aufheizzeit, in der das Synthesesol auf die Kristallisationstemperatur erwärmt wird abhängt.

Unter den vorgegebenen Bedingungen des erfindungsgemäßen Verfahrens beträgt die Kristallisationszeit üblicherweise weniger als 3 Tage, vorzugsweise höchstens 24 h. Die Kristalle werden durch Filtrieren, Zentrifugieren und Dekandieren von der Mutterlauge abgetrennt und mit einer geeigneten Waschflüssigkeit, vorzugsweise Wasser, gewaschen. Anschließend werden die Kristalle ggf. getrocknet und zur Entfernung des Templats bei einer Temperatur zwischen 400°C und 1.000°C, vorzugsweise zwischen 500°C und 750°C calciniert.

Die erfindungsgemäßen kristallinen titanhaltigen Zeolithe werden in Pulverform erhalten. Für ihre Anwendung als Oxydationskatalysatoren können sie wahlweise durch bekannte Verfahren zur Verformung von Pulverkatalysatoren wie beispielsweise Granulation, Sprühtrocknen, Sprühgranulation oder Extrusion in eine zur Anwendung geeignete Form wie beispielsweise Mikrogranulate, Kugeln, Tabletten, Vollzylinder, Hohlzylinder oder Wabenkörper gebracht werden.

Der erfindungsgemäß hergestellte titanhaltige Zeolith kann als Katalysator bei Oxydationsreaktionen mit $H_2O_2$ eingesetzt werden. Insbesondere kann der erfindungsgemäße titanhaltige Zeolith als Katalysator bei der Epoxidierung von Olefinen mittels wäßrigem Wasserstoffperoxid in einem mit Wasser mischbaren Lösungsmittel verwendet werden.

Mit dem erfindungsgemäßen Verfahren kann mit vergleichsweise einfachen Mitteln die Aktivität des durch das erfindungsgemäße Verfahren hergestellten titanhaltigen Zeolithen, insbesondere in Epoxidierungsreaktionen von Olefinen weiter gesteigert werden.

Die vorliegende Erfindung soll nun anhand der Beispiele genauer erläutert werden.

Beispiel 1

In einem mit Stickstoff inertisierten 10-1-Autoklaven werden 3.415,2 g Tetraethylorthosilicat vorgelegt, unter Rühren 93,5 g Tetraethylorthotitanat zugegeben und die resultierende Mischung auf ca. 1,0°C abgekühlt. Anschließend wird bei dieser Temperatur unter Rühren innerhalb von etwa 5 h eine Lösung aus 1.417 g Tetra-n-propylammoniumhydroxid (TPAOH 40 Gew.-%ige Lösung) und 3,075 g entionisiertem Wasser mittels einer Schlauchpumpe zudosiert. Um die Hydrolyse zu vervollständigen und das gebildete Ethanol abzudestillieren, wurde die Reaktionsmischung zunächst auf ca. 80°C und innerhalb von etwa 3 h auf max. 95°C erhitzt. Das dabei abdestillierte Ethanol-Wasser-Azeotrop wird durch das gleiche Volumen entionisiertes Wasser ersetzt.

Anschließend wurde das Synthesesol innerhalb von 150 min auf 175°C erhitzt und bei dieser Temperatur für die Dauer von 120 min gehalten. Nach Abkühlen der resultierenden Titansilicalit-1-Suspension wurde der gebildete Feststoff durch Zentrifugieren von der stark basischen noch TPAOH-haltigen Mutterlauge abgetrennt, gewaschen, über Nacht bei 120°C getrocknet und anschließend in Luftatmosphäre bei 550°C 5 h in einem Muffelofen calciniert.

Die Produkteigenschaften wie Titangehalt im resultierenden Titansilicalit sowie die Aktivitätskennzahl sind in Tabelle 1 angegeben.

Die Aktivitätskennzahl wurde wie folgt gemessen:

In einem thermostatisierten Laborautoklav mit Begasungsrührer wurden dann 1,0 g des im Vergleichsbeispiel 1 hergestellten Titansilicalitkatalysators in 300 ml Methanol bei 40°C unter Propylenatmosphäre vorgelegt und das Lösungsmittel bei 3 bar Überdruck mit Propylen gesättigt. Dann werden unter Rühren 13,1 g 30 Gew.-% wäßrige Wasserstoffperoxidlösung in einer Portion zugegeben und die Reaktionsmischung bei 40°C und 3 bar gehalten, wobei über einen Druckregler Propylen nachdosiert wurde, um den Verbrauch durch die Reaktion auszugleichen. In regelmäßigen Abständen wurden über ein Filter Proben entnommen und der Wasserstoffperoxidgehalt der Reaktionsmischung durch Redoxtitration mit Cer(IV)sulfat-Lösung bestimmt. Die Auftragung von $\ln(c/c_0)$ gegen die Zeit t, wobei c die gemessene $H_2O_2$-Konzentration zum Zeitpunkt t und $c_0$ die berechnete $H_2O_2$-Konzentration zu Beginn der Reaktion ist, ergibt eine Gerade. Aus der Steigerung der Geraden wurde mit der Beziehung $\frac{dc}{dt} = k.c.c_{kat}$, worin $C_{kat}$ für die

Katalysatorkonzentration in kg Katalysator je kg Reaktionsmischung steht, die Aktivitätskennzahl bestimmt.

Beispiele 2 und 3

Das Beispiel 1 wurde mit identischen Molverhältnissen der Ausgangsverbindung entsprechend wiederholt. Lediglich die Ansatzgröße und die Aufheizzeit wurden variiert. Ansatzgröße, Aufheizzeit und die Produkteigenschaften des resultierenden Titansilicalitkatalysators sind in Tabelle 1 angegeben.

Vergleichsbeispiel 1

In einem trockenen mit Stickstoff inertisierten 300-l-Reaktor mit Destiallationskolonne wurden 128,1 kg Tetraethylorthosilicat vorgelegt und unter Rühren 3,51 kg Tetraethylorthotitanat zugegeben. Unter Spülen mit Stickstoff wurde der Reaktorinhalt auf 0°C abgekühlt. Anschließend wurde innerhalb von 6,5 h eine Mischung aus 53,12 kg Tetra-n-propylammoniumhydroxidlösung (TPAOH, 40 Gew.-%ige Lösung) und 100 kg entionisiertem Wasser zudosiert. Um die Hydrolyse zu vervollständigen wurde die Reaktionsmischung auf zunächst 77°C und innerhalb von 5,5 h auf 96°C erhitzt. Das dabei abdestillierte Ethanol/Wasser-Azeotrop wurde durch das gleiche Volumen entionisiertes Wasser ersetzt.
Das Synthesesol wurde anschließend in einem 300-l-Autoklaven gepumpt, unter Rühren innerhalb von 8 h auf 175°C aufgeheizt und bei dieser Temperatur 1 h gehalten. Nach Abkühlen der resultierenden Titansilicalitsuspension wurde sie wie in Beispiel 1 beschrieben aufgearbeitet.
Die wesentlichsten Prozeßparameter und die Eigenschaften des resultierenden Produkts sind in Tabelle 1 angegeben.

Vergleichsversuche 2 und 3

Vergleichsversuche 2 und 3 wurden entsprechend des Vergleichsversuchs 1 mit identischem molaren Verhältnis der Ausgangsverbindung durchgeführt. Lediglich die Ansatzgröße und Aufheizzeit waren wie in Tabelle 1 angegeben. Die Eigenschaften der resultierenden Titansilicalitprodukte sind ebenfalls in Tabelle 1 zusammengefaßt.

Tabelle 1

| Versuch | Ansatzgröße | Aufheizzeit | Produkteigenschaften | |
|---|---|---|---|---|
| | (l) | (h) | Titangehalt (Gew. -% $TiO_2$) | Aktivitätskennzahl ($min^{-1}$) |
| 1 | 10 | 2,5 | 2,8 | 30,5 |
| 2 | 1 | 1,5 | 3,2 | 31,6 |
| 3[a] | 1 | 1,5 | 2,9 | 30,8 |
| 4[b] | 1 | 2,5 | 3,0 | nb |
| V1 | 300 | 8 | 2,5 | 26,9 |
| V2 | 300 | 8 | 2,4 | 22,5 |
| V3[b] | 1 | 8 | 2,5 | 22,4 |

a) Synthesesol im 300-l-Maßstab hergestellt.

b) Synthesesol im 2-l-Maßstab hergestellt und dann geteilt.

Die Primärkristallitgröße der nach den Beispielen gemäß der vorliegenden Erfindung sowie nach den Vergleichsbeispielen hergestellten Titansilicalite liegen unabhängig von Ansatzgröße und Aufheizzeit im Bereich von 0,3 bis 0,4 $\mu$m.
Vergleicht man die Beispiele gemäß der vorliegenden Erfindung mit den Vergleichsbeispielen erkennt man, daß die Aufheizzeit einen direkten Einfluß auf die Aktivität des resultierenden Katalysators hat. Ohne sich an eine Theorie binden zu wollen, wird aus den experimentellen Ergebnissen deutlich, daß die Zunahme des Gehalts des in das Kristallgitter eingebauten Titans mit der abnehmenden Aufheizzeit korreliert. Diese Korrelation könnte ein Grund für die überraschenderweise gefundene Zunahme der Katalysatoraktivität mit abnehmender Zeit, in der das Synthesesol auf Kristallisationstemperatur gebracht wird, sein.

**Patentansprüche**

1. Verfahren zur Herstellung eines titanhaltigen Zeolithen durch

   a) Zusammengeben einer hydrolisierbaren Siliciumverbindung und einer hydrolisierbaren Titanverbindung,
   b) Zugeben einer basischen quartären Ammoniumverbindung in wäßrigem Medium zu der Mischung aus a) und Hydrolisieren der Reaktionsmischung bei einer Temperatur im Bereich von 0°C - 100° zur Ausbildung eines Synthesesols, anschließend
   c) Erwärmen der Synthesesols auf eine Temperatur im Bereich von 150°C bis 190°C und
   d) Kristallisieren des Synthesesols bei dieser Temperatur,

   **dadurch gekennzeichnet**, daß
   die Aufheizzeit in Schritt c) weniger als 240 Minuten beträgt.

2. Verfahren nach Anspruch 1,
   **dadurch gekennzeichnet**, daß
   die Aufheizzeit 60 - 180 Minuten, vorzugsweise 80 - 120 Minuten beträgt.

3. Verfahren nach einem der vorstehenden Ansprüche,
   **dadurch gekennzeichnet**, daß
   die hydrolisierbare Siliciumverbindung ein Tetraalkylorthosilikat, vorzugsweise Tetraethylorthosilikat ist und die hydrolisierbare Titanverbindung ein Tetraalkylorthotitanat, vorzugsweise Tetraethylorthotitanat ist.

4. Verfahren nach Anspruch 3,
   **dadurch gekennzeichnet**, daß
   nach Schritt b) und vor Schritt c) das Synthesesol auf eine Temperatur von 75°C - 100°C für eine Zeitdauer von 120 - 200 Minuten erwärmt und der entstehende Alkohol abdestilliert wird, um die Hydrolyse der hydrolisierbaren Titan- und Siliciumverbindungen zu unterstützten und fakultativ das Volumen des abdestillierten Alkohols zumindest teilweise durch Zugabe von Wasser zum Synthesesol ersetzt wird.

5. Verfahren nach einem der vorstehenden Ansprüche,
   wobei die quartäre Ammoniumverbindung ein Tetraalkylammoniumhydroxid, vorzugsweise Tetra-n-propylammoniumhydroxid ist.

6. Verfahren nach einem der vorstehen Ansprüche,
   wobei der titanhaltige Zeolith abgetrennt, getrocknet und kalziniert wird.

7. Verfahren zur Epoxidierung von Olefinen durch Umsetzen von Olefin mit wäßrigem Wasserstoffperoxid in einem mit Wasser mischbaren Lösungsmittel in Gegenwart eines titanhaltigen Zeolithen als Katalysator, der durch das Verfahren nach Anspruch 6 erhältlich ist.

8. Verwendung eines titanhaltigen Zeolithen, der nach dem Verfahren nach Anspruch 6 erhältlich ist, als Katalysator für die Epoxidierung von Olefinen mit Wasserstoffperoxid.

# EP 1 129 992 A1

**Europäisches Patentamt**

## EUROPÄISCHER RECHERCHENBERICHT

Nummer der Anmeldung

EP 00 10 4325

### EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.7) |
|---|---|---|---|
| A | FR 2 471 950 A (SNAM PROGETTI) 26. Juni 1981 (1981-06-26) * Ansprüche 1-3,6-9 * * Beispiel 1 * | 1,3,6-8 | C01B37/00 B01J29/04 C07D301/12 |
| D,A | & US 4 410 501 A 18. Oktober 1983 (1983-10-18) --- | | |
| A | THANGARAJ ET AL.: "STUDIES ON THE SYNTHESIS OF TITANIUM SILICALITE, TS-1" ZEOLITES, US, BUTTERWORTH-HEINEMANN, Bd. 12, Nr. 8, 1. November 1992 (1992-11-01), Seiten 943-950, XP000198990 * das ganze Dokument * --- | 1,3-8 | |
| A | A. THANGARAJ ET AL.: "CATALYTIC PROPERTIES OF TITANIUM SILICALITES. I. SYNTHESIS AND CHARACTERIZATION OF TITANIUM-RICH ZEOLITES WITH MFI STRUCTURE" JOURNAL OF CATALYSIS., Bd. 130, 1991, Seiten 1-8, XP002143172 * das ganze Dokument * ----- | 1,3-6 | |

**RECHERCHIERTE SACHGEBIETE (Int.Cl.7)**

C01B

Der vorliegende Recherchenbericht wurde für alle Patentansprüche erstellt

| Recherchenort | Abschlußdatum der Recherche | Prüfer |
|---|---|---|
| DEN HAAG | 21. Juli 2000 | Rigondaud, B |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

& : Mitglied der gleichen Patentfamilie, übereinstimmendes Dokument

EPO FORM 1503 03.82 (P04C03)

# EP 1 129 992 A1

## ANHANG ZUM EUROPÄISCHEN RECHERCHENBERICHT
## ÜBER DIE EUROPÄISCHE PATENTANMELDUNG NR.

EP 00 10 4325

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

21-07-2000

| Im Recherchenbericht angeführtes Patentdokument | Datum der Veröffentlichung | Mitglied(er) der Patentfamilie | | Datum der Veröffentlichung |
|---|---|---|---|---|
| FR 2471950 A | 26-06-1981 | IT | 1127311 B | 21-05-1986 |
| | | AR | 231610 A | 31-01-1985 |
| | | AT | 385022 B | 10-02-1988 |
| | | AT | 586880 A | 15-07-1987 |
| | | AU | 537263 B | 14-06-1984 |
| | | AU | 6451380 A | 02-07-1981 |
| | | BE | 886812 A | 22-06-1981 |
| | | BG | 39637 A | 15-07-1986 |
| | | BR | 8008379 A | 07-07-1981 |
| | | CA | 1155830 A | 25-10-1983 |
| | | CH | 645599 A | 15-10-1984 |
| | | CS | 257253 B | 15-04-1988 |
| | | DD | 155420 A | 09-06-1982 |
| | | DE | 3047798 A | 22-10-1981 |
| | | DK | 493980 A,B, | 22-06-1981 |
| | | ES | 498480 D | 16-11-1981 |
| | | ES | 8200823 A | 16-02-1982 |
| | | GB | 2071071 A,B | 16-09-1981 |
| | | GR | 72832 A | 07-12-1983 |
| | | HU | 183272 B | 28-04-1984 |
| | | IL | 61654 A | 30-03-1984 |
| | | IN | 154032 A | 08-09-1984 |
| | | JP | 1042889 B | 18-09-1989 |
| | | JP | 1561240 C | 31-05-1990 |
| | | JP | 56096720 A | 05-08-1981 |
| | | LU | 83014 A | 23-07-1981 |
| | | NL | 8006939 A,B, | 16-07-1981 |
| | | NO | 803859 A,B, | 22-06-1981 |
| | | PH | 17732 A | 21-11-1984 |
| | | PL | 228600 A | 07-08-1981 |
| | | PT | 72248 A,B | 01-01-1981 |
| | | RO | 81245 A | 01-06-1983 |
| | | SE | 447818 B | 15-12-1986 |
| | | SE | 8008961 A | 22-06-1981 |
| | | RU | 2076775 C | 10-04-1997 |
| | | TR | 21473 A | 04-07-1984 |
| | | US | 4410501 A | 18-10-1983 |
| | | YU | 316780 A | 30-09-1983 |
| | | ZA | 8007233 A | 27-01-1982 |
| | | ZM | 11280 A | 21-12-1981 |

EPO FORM P0461

Für nähere Einzelheiten zu diesem Anhang : siehe Amtsblatt des Europäischen Patentamts, Nr.12/82

7